# EUROPEAN PATENT APPLICATION

(11) **EP 4 089 100 A1**
(43) Date of publication of application: **16.11.2022**
(21) Application number: 21173824.0
(22) Date of filing: 14.05.2021
(51) Int. Cl.: C07J 9/00, C07J 31/00, C07J 41/00, C07J 71/00

(54) **NOVEL SYNTHESIS OF CHOLESTEROL**

(71) Applicant: CordenPharma International GmbH, 68723 Plankstadt (DE); Otto-von-Guericke-Universität Magdeburg, 39106 Magdeburg (DE)
(72) Inventor: SCHINZER, Dieter, 38108 Braunschweig (DE); SPIE , Oliver, 21029 Hamburg (DE); MUNT, Maxim, 39104 Magdeburg (DE); INDOLESE, Adriano, 22301 Hamburg (DE); ROUX, Lionel, 68510 Uffheim (FR)
(74) Representative: Schulz Junghans Patentanwälte PartGmbB

(57) **Abstract**

The invention relates to a synthesis of cholesterol; a ring opening step of the compound of formula (I) 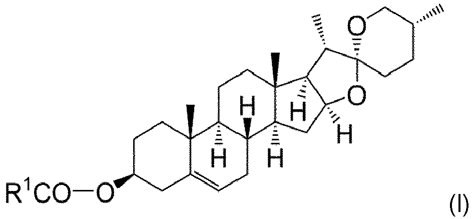 and subsequent activation and reduction step yielding cholesterol. The inventions relates also to intermediates achieved during said synthesis.

## Description

The present invention relates to the synthesis method of cholesterol and intermediates of cholesterol.

### Background of the Invention

Cholesterol is found in all tissues of the animal body. It is particularly prevalent in the hippocampus, cerebral cortex and spinal cord as well as in the pancreas and in the kidneys. Cholesterol plays important roles in these tissues: it serves as a building block of cell membranes and is a precursor of various biologically important molecules. As a component of a cellular membrane, cholesterol modulates the fluidity of the membranes. It also assists formation of lipid rafts to facilitate cell signalling.

Cholesterol is used as a precursor for several biochemical pathways. Most prominently, cholesterol is a precursor for the synthesis of all steroid hormones, including cortisol, progesterone, estrogen testosterone as well as derivatives thereof. Cholesterol also constitutes a vitamin D precursor in the calcium metabolism.

Cholesterol is also used widely used in numerous industries, most prominently in cosmetics industry as a component of the skin care and make up products. In these products, cholesterol serves as an emulsifier to prevent phase separation in oily combination such as facial creams and skin conditioners. In these compositions, cholesterol is also an emollient and polymer stabilizer. Additionally, cholesterol is added to various compositions as a viscosity regulator.

In recent year cholesterol biological properties of controlling formation and fluidity of lipid membranes have been employed in drug delivery systems. Most prominently, cholesterol plays an important role in assembly of lipid nanoparticles (LNPs) and their escape from endosomes. The LNPs are critical for the delivery of mRNA vaccines such as SARS-Cov2 vaccine that delivers mRNA encoding viral spike protein.

Currently, industrial quantities of cholesterol are obtained from animal tissues. The most prominent source of commercial cholesterol is cattle spinal cords wherein cholesterol is extracted from the tissue with petroleum ether. For instance, patent US 2371467 A discloses a method of extracting cholesterol from animal nerve tissue with ethylene dichloride. Another common source of cholesterol is wool grease, wherein cholesterol is extracted with alcohol. For example, US 2650929 A discloses extraction of cholesterol by dissolving wool grease in methanol and subsequent crystallization of cholesterol. However, due to heavy reliance of various industries on constant supply of cholesterol a development of novel production methods is of interest.

The existing methods of cholesterol extraction provide large quantities of the substance. However, there are several problems associated with them: i) they require large amounts of animal material. Cattle cultivation contributes about 25% of global methane emission and with global effort to reduce greenhouse emission, it is possible that in near future cattle nerve tissue will become scarce and therefore more expensive; ii) extraction process generates significant amounts of waste products, as cholesterol naturally is accompanied by isocholesterol, a compound that has minor industrial use; iii) cholesterol produced from animal sources carries a risk of contamination with Transmissible Spongiform Encephalopathies-causing prions.

Thus, a non-animal source for the production of cholesterol, such as plant-derived diosgenin, is important.

Diosgenin is a phytosteroid saponin that is a product of hydrolysis of saponins extracted from the tubers of wild yam *Dioscorea.*

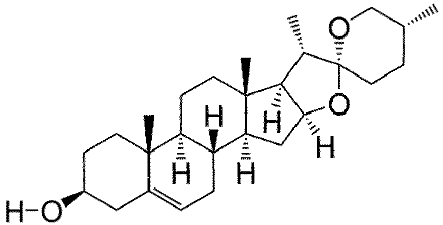

The present invention provides a solution to above mentioned problems by providing a cheaper and safer alternative to animal sources for cholesterol. It is accomplished by disclosing a method to chemically synthesize cholesterol from plant-derived substance - diosgenin.

In a novel series of reactions the 3-hydroxyl group of diosgenin is protected by acylation, the tetrahydrofuran and tetrahydropyran rings are opened, newly obtained hydroxyl groups are activated and the resulting compound is reduced, yielding cholesterol.

### Summary of the Invention

The first aspect of the invention in the synthesis method of cholesterol comprising:
a) a ring opening step, wherein the compound of formula (I) 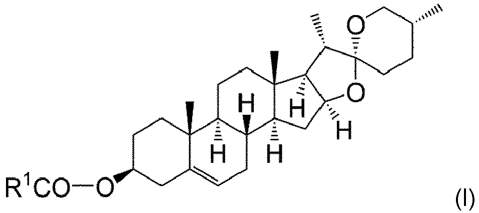 is reacted in a Clemmensen reaction or a variant thereof, yielding compound of formula (II); 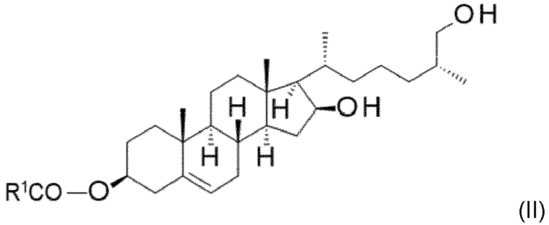
b) an activation step with SOCI2, PBr₃ R⁴SO₂X, yielding a compound of formula (III) preferably (IIIa); 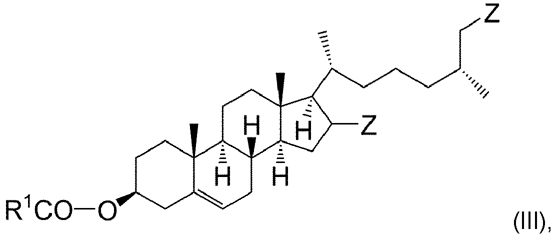 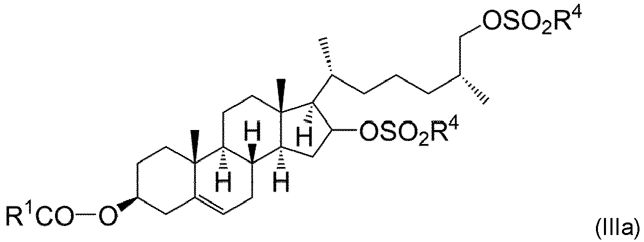
c) a reduction step, wherein compound (III) is reacted with a reducing agent, yielding cholesterol (IV) 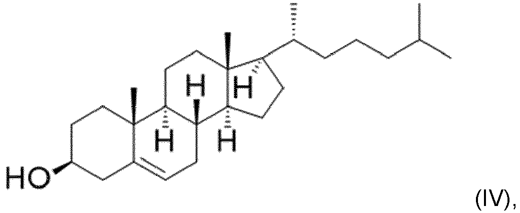 wherein
   - R¹ is selected from unsubstituted or substituted alkyl, amino acid, cyclic alkyl, particularly a C₂-C₆ cyclic alkyl, or alkyl-R², heterocycle, an aromatic or heteroaromatic group, particularly R¹ is selected from unsubstituted or substituted alkyl, amino acid, cyclic alkyl, particularly a C₂-C₆ cyclic alkyl, or alkyl-R², more particularly from unsubstituted or substituted alkyl, amino acid or alkyl-R²,
   - R² is selected from H, -OH, -CN, -NH₂, -NHR³ or -NR³₂,
   - R³ is an unsubstituted or substituted C₁-C₆ alkyl,
   - R⁴ is a substituted or unsubstituted alkyl, cyclic alkyl or aromatic group,
   - X is a leaving group or OSO₂R⁴
   - Z is -CI, -Br or -OSO₂R⁴, preferably -OSO₂R⁴.

A second aspect of the invention are intermediate substrates of the above reaction sequence in for of compound of general formula (I), formula (II) or formula (III), particularly formula (II) or formula (III), 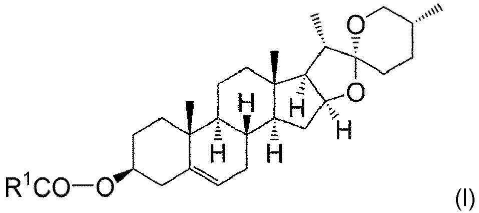
- wherein R¹ has the same definition as above with the exception that R¹ is not an unsubstituted alkyl. 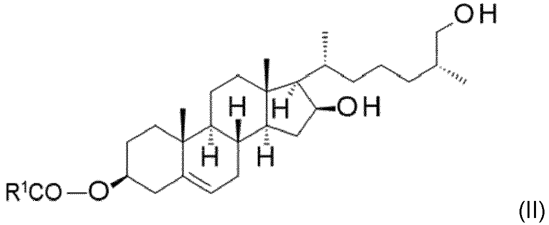
- wherein R¹ has the same definition as above, 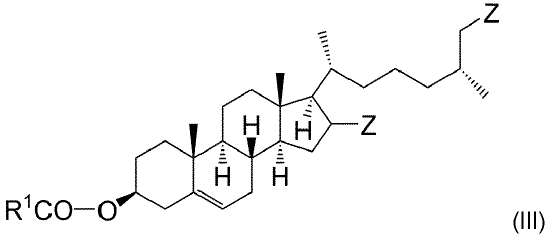
- wherein R¹ and Z have the same definition as above.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art,

The term *C₁-C₁₀ alkyl* in the context of the present specification relates to a saturated linear or branched hydrocarbon having between 1 and 10 carbon atoms, wherein in certain embodiments one carbon-carbon bond may be unsaturated and one CH₂ moiety may be exchanged for oxygen (ether bridge) or nitrogen (NH, or NR with R being methyl, ethyl, or propyl; amino bridge). Non-limiting examples for a C₁-C₁₀ alkyl are methyl, ethyl, propyl, prop-2-enyl, n-butyl, 2-methylpropyl, *tert-butyl,* but-3-enyl, prop-2-inyl and but-3-inyl. In certain embodiments, a C₁-C₁₀ alkyl is a methyl, ethyl, propyl or butyl moiety.

A *C₁-C₆ alkyl* in the context of the present specification relates to a saturated linear or branched hydrocarbon having 1, 2, 3, 4, 5 or 6 carbon atoms, wherein one carbon-carbon bond may be unsaturated and/or one CH₂ moiety may be exchanged for oxygen (ether bridge) or nitrogen (NH, or NR with R being methyl, ethyl, or propyl; amino bridge). Non-limiting examples for a C₁-C₆ alkyl include the examples given for C₁-C₄ alkyl above, and additionally 3-methylbut-2-enyl, 2-methylbut-3-enyl, 3-methylbut-3-enyl, n-pentyl, 2-methylbutyl, 3-methylbutyl, 1,1-dimethylpropyl, 1,2-dimethylpropyl, 1,2-dimethylpropyl, pent-4-inyl, 3-methyl-2-pentyl, and 4-methyl-2-pentyl. In certain embodiments, a C₅ alkyl is a pentyl or cyclopentyl moiety and a C₆ alkyl is a hexyl or cyclohexyl moiety. The same applies to C₁-C₃ alkyl.

The term *C₄-C₇ cycloalkyl* in the context of the present specification relates to a saturated hydrocarbon ring having 4, 5, 6 or 7 carbon atoms, wherein in certain embodiments, one carbon-carbon bond may be unsaturated and/or one CH₂ moiety may be exchanged for oxygen (ether bridge) or nitrogen (NH, or NR with R being methyl, ethyl, or propyl; amino bridge). Non-limiting examples of a C₄-C₇ cycloalkyl moiety include cyclobutyl (-C₄H₇), cyclopentenyl (C₅H₉), and cyclohexenyl (C₆H₁₁) moieties. In certain embodiments, a cycloalkyl is substituted by one C₁ to C₄ unsubstituted alkyl moiety. In certain embodiments, a cycloalkyl is substituted by more than one C₁ to C₄ unsubstituted alkyl moieties. A heterocycle is an cycloalkyl that comprises one or several nitrogen, oxygen and/or sulphur atoms. An heterocycle in the context of the specification additionally may be substituted by one or more alkyl groups.

The term *substituted alkyl* in its broadest sense refers to an alkyl as defined above in the broadest sense, which is covalently linked to an atom that is not carbon or hydrogen, particularly to an atom selected from N, O, F, B, Si, P, S, CI, Br and I, which itself may be -if applicable- linked to one or several other atoms of this group, or to hydrogen, or to an unsaturated or saturated hydrocarbon (alkyl or aryl in their broadest sense). In a narrower sense, *substituted alkyl* refers to an alkyl as defined above in the broadest sense that is substituted in one or several carbon atoms by groups selected from amine NH₂, alkylamine NHR, imide NH, alkylimide NR, amino(carboxyalkyl) NHCOR or NRCOR, hydroxyl OH, oxyalkyl OR, oxy(carboxyalkyl) OCOR, carbonyl O and its ketal or acetal (OR)₂, nitril CN, isonitril NC, cyanate CNO, isocyanate NCO, thiocyanate CNS, isothiocyanate NCS, fluoride F, choride CI, bromide Br, iodide I, phosphonate PO₃H₂, PO₃R₂, phosphate OPO₃H₂ and OPO₃R₂, sulfhydryl SH, suflalkyl SR, sulfoxide SOR, sulfonyl SO₂R, sulfanylamide SO₂NHR, sulfate SO₃H and sulfate ester SO₃R, wherein the R substituent as used in the current paragraph, different from other uses assigned to R in the body of the specification, is itself an unsubstituted or substituted C₁ to C₁₂ alkyl in its broadest sense, and in a narrower sense, R is methyl, ethyl or propyl unless otherwise specified.

The term *amino substituted alkyl or hydroxyl* substituted alkyl refers to an alkyl according to the above definition that is modified by one or several amine or hydroxyl groups NH₂, NHR, NR₂ or OH, wherein the R substituent as used in the current paragraph, different from other uses assigned to R in the body of the specification, is itself an unsubstituted or substituted C₁ to C₁₂ alkyl in its broadest sense, and in a narrower sense, R is methyl, ethyl or propyl unless otherwise specified. An alkyl having more than one carbon may comprise more than one amine or hydroxyl. Unless otherwise specified, the term "substituted alkyl" refers to alkyl in which each C is only substituted by at most one amine or hydroxyl group, in addition to bonds to the alkyl chain, terminal methyl, or hydrogen.

The term *carboxyl substituted alkyl* refers to an alkyl according to the above definition that is modified by one or several carboxyl groups COOH, or derivatives thereof, particularly carboxylamides CONH₂, CONHR and CONR₂, or carboxylic esters COOR, with R having the meaning as laid out in the preceding paragraph and different from other meanings assigned to R in the body of this specification.

Non-limiting examples of amino-substituted alkyl include -CH₂NH₂, -CH₂NHMe, -CH₂NHEt, -CH₂CH₂NH₂, -CH₂CH₂NHMe, -CH₂CH₂NHEt, -(CH₂)₃NH₂, -(CH₂)₃NHMe, -(CH₂)₃NHEt, -CH₂CH(NH₂)CH₃, -CH₂CH(NHMe)CH₃, -CH₂CH(NHEt)CH₃, -(CH₂)₃CH₂NH₂, -(CH₂)₃CH₂NHMe, -(CH₂)₃CH₂NHEt, -CH(CH₂NH₂)CH₂CH₃, -CH(CH₂NHMe)CH₂CH₃, -CH(CH₂NHEt)CH₂CH₃, -CH₂CH(CH₂NH₂)CH₃, -CH₂CH(CH₂NHMe)CH₃, -CH₂CH(CH₂NHEt)CH₃, -CH(NH₂)(CH₂)₂NH₂, -CH(NHMe)(CH₂)₂NHMe, -CH(NHEt)(CH₂)₂NHEt, -CH₂CH(NH₂)CH₂NH₂, -CH₂CH(NHMe)CH₂NHMe, -CH₂CH(NHEt)CH₂NHEt, -CH₂CH(NH₂)(CH₂)₂NH₂, -CH₂CH(NHMe)(CH₂)₂NHMe, -CH₂CH(NHEt)(CH₂)₂NHEt, -CH₂CH(CH₂NH₂)₂, -CH₂CH(CH₂NHMe)₂, -CH₂NMe₂, - CH₂NMeEt, -CH₂CH₂NMe₂, -CH₂CH₂NMeEt, -(CH₂)₃NMe₂, -(CH₂)₃NMeEt, -CH₂CH(NMe₂)CH₃, -CH₂CH(NMeEt)CH₃, -(CH₂)₃CH₂NMe#2, -(CH₂)₃CH₂NMeEt, - - CH(CH₂NMe₂)CH₂CH₃, -CH(CH₂NMeEt)CH₂CH₃, -CH₂CH(CH₂NMe₂)CH₃, -CH₂CH(CH₂NMeEt)CH₃, -CH(NMe₂)(CH₂)₂NMe₂, -CH(NMeEt)(CH₂)₂NMeEt, - CH₂CH(NMe₂)CH₂NMe₂, -CH₂CH(NMeEt)CH₂NMeEt, -CH₂CH(NMe₂)(CH₂)₂NMe₂, -CH₂CH(NMeEt)(CH₂)₂NMeEt, and -CH₂CH(CH₂NHEt)₂ for terminal moieties and -CH₂CHNH₂-, -CH₂CHNHMe-, -CH₂CHNHEt- for an amino substituted alkyl moiety bridging two other moieties.

Non-limiting examples of hydroxy-substituted alkyl include -CH₂OH, -(CH₂)₂OH, -(CH₂)₃OH, -CH₂CH(OH)CH₃, -(CH₂)₄OH, -CH(CH₂OH)CH₂CH₃, -CH₂CH(CH₂OH)CH₃, -CH(OH)(CH₂)₂OH, -CH₂CH(OH)CH₂OH, -CH₂CH(OH)(CH₂)₂OH and -CH₂CH(CH₂OH)₂ for terminal moieties and -CHOH-, -CH₂CHOH-, -CH₂CH(OH)CH₂-, -(CH₂)₂CHOHCH₂-, - CH(CH₂OH)CH₂CH₂-, -CH₂CH(CH₂OH)CH₂-, -CH(OH)(CH₂CHOH-, -CH₂CH(OH)CH₂OH, - CH₂CH(OH)(CH₂)₂OH and -CH₂CHCH₂OHCHOH- for a hydroxyl substituted alkyl moiety bridging two other moieties.

The term *halogen-substituted alkyl* refers to an alkyl according to the above definition that is modified by one or several halogen atoms selected (independently) from F, CI, Br, I.

The term *fluoro substituted alkyl* refers to an alkyl according to the above definition that is modified by one or several fluoride groups F. Non-limiting examples of fluoro-substituted alkyl include -CH₂F, -CHF₂, -CF₃, -(CH₂)₂F, -(CHF)₂H, -(CHF)₂F, -C₂F₅, -(CH₂)₃F, -(CHF)₃H, - (CHF)₃F, -C₃F₇, -(CH₂)₄F, -(CHF)₄H, -(CHF)₄F and -C₄F₉.

Non-limiting examples of hydroxyl- and fluoro-substituted alkyl include -CHFCH₂OH, - CF₂CH₂OH, -(CHF)₂CH₂OH, -(CF₂)₂CH₂OH, -(CHF)₃CH₂OH, -(CF₂)₃CH₂OH, -(CH₂)₃OH, -CF₂CH(OH)CH₃, -CF₂CH(OH)CF₃, -CF(CH₂OH)CHFCH₃, and -CF(CH₂OH)CHFCF₃.

The term *aryl* in the context of the present specification relates to a cyclic aromatic C₅-C₁₀ hydrocarbon that may comprise a heteroatom (e.g. N, O, S). Examples of aryl include, without being restricted to, phenyl and naphthyl, and any heteroaryl. A heteroaryl is an aryl that comprises one or several nitrogen, oxygen and/or sulphur atoms. Examples for heteroaryl include, without being restricted to, pyrrole, thiophene, furan, imidazole, pyrazole, thiazole, oxazole, pyridine, pyrimidine, thiazin, quinoline, benzofuran and indole. An aryl or a heteroaryl in the context of the specification additionally may be substituted by one or more alkyl groups.

An *alkyl aryl* in the context of the present specification relates to an alkyl group substituted by an aryl moiety. Particular examples are methylphenyl, ethylphenyl, propylphenyl, butylphenyl and their higher homologues. A substituted alkyl aryl may be substituted by the substituent indicated on the alkyl part, if chemically feasible, or on the aryl part of the moiety.

A *carboxylic ester* is a group -CO₂R, with R being defined further in the description. A carboxylic amide is a group -CONHR, with R being defined further in the description.

The term *leaving group* relates to a functional group within a molecule that exerts a -M and/or -I effect and can thus easily be cleaved off, wherein the binding electron pair remains with the leaving group after cleavage.

### Detailed Description of the Invention

The first aspect of the invention in the synthesis method of cholesterol comprising:
a ring opening step, wherein the compound of formula (I) 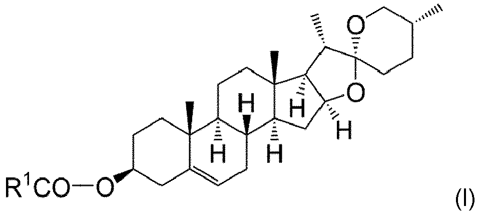
is reacted in a Clemmensen reaction or a variant thereof, yielding compound of formula (II).

This reaction allows for efficient opening of both tetrahydrofuran and tetrahydropyran rings, yielding long, saturated, branched side chain, that is critical for the subsequent synthesis of cholesterol.

Subsequently the compound of formula (II) 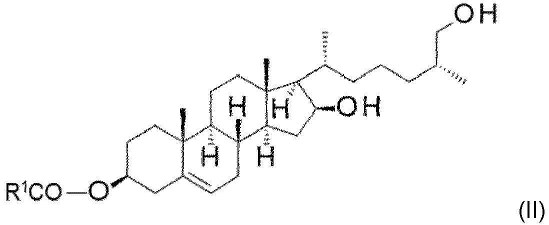 is reacted in an activation step with an activating agent, selected from SOCl₂, PBr₃ or R⁴SO₂X, yielding a compound of formula (III), preferably (IIIa).

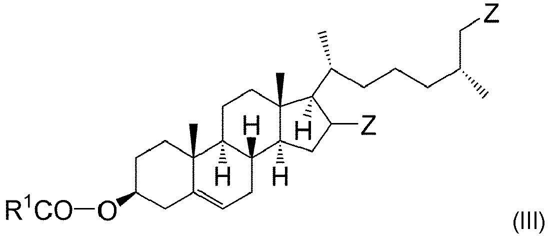

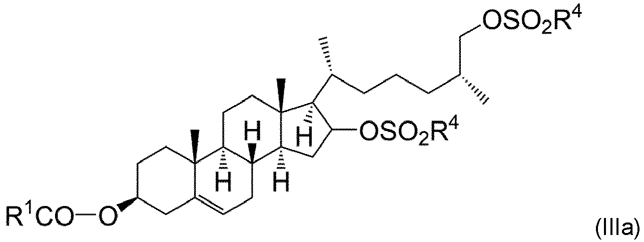

The activation allows for a subsequent reduction of the two hydroxyl groups position.

A subsequent reduction step with a reducing agent yielding cholesterol (IV).

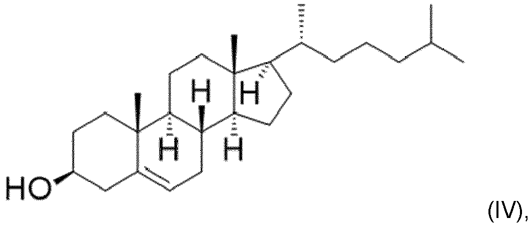

The reduction step allows the reduction of the activated hydroxyl positions as well as the deprotection of the protected R¹CO-O- position in one synthesis step.

The entire sequence of reactions enables synthesis of cholesterol from plant-based chemical compound with a high yield and minimal waste production. In the above-described sequence
- R¹ is selected from unsubstituted or substituted alkyl, amino acid, cyclic alkyl, particularly a C₂-C₆ cyclic alkyl, or alkyl-R², heterocycle, an aromatic or heteroaromatic group, particularly R¹ is selected from unsubstituted or substituted alkyl, amino acid, cyclic alkyl, particularly a C₂-C₆ cyclic alkyl, or alkyl-R², more particularly from unsubstituted or substituted alkyl, amino acid or alkyl-R²,
- R² is selected from H, -OH, -CN, -NH₂, -NHR³ or -NR³₂,
- R³ is an unsubstituted or substituted C₁-C₆ alkyl,
- R⁴ is a substituted or unsubstituted alkyl, cyclic alkyl or aromatic group,
- X is a leaving group or OSO₂R⁴
- Z is -CI, -Br or -OSO₂R⁴, preferably -OSO₂R⁴.

The method disclosed in the current invention enables efficient conversion of a plant-based diosgenin to cholesterol. Addition of an organic moiety to the 3-hydroxyl group protects it from reacting with any mild reactants in any subsequent steps, but it is deprotected in the last reduction step. This protection enables opening of tetrahydropyran and tetrahydrofuran rings in a previously unattainable way, leading to formation of a steroid nucleus with a free hydroxyl group and a long, saturated and branched side chain capped with a free hydroxyl group at the pentane ring. The free hydroxyl groups are then activated, that enables removal of these groups while retaining the original hydroxyl group. This solution allows easy and efficient conversion of diosgenin into cholesterol.

In an embodiment, the compound I is achieved by a reaction of diosgenin V 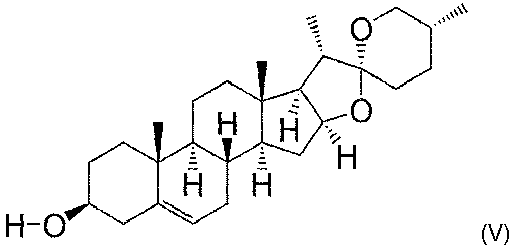 with a compound of formula R¹COY, wherein R¹ is defined as above and Y is a leaving group.

The protecting group R¹CO allows for a protection during the ring opening reaction and a deprotection during the reduction step of the activated hydroxyl groups of the ring opening sequence. The protecting group is chosen in such a way that it allows for a good protection while retaining a suitable solubility during the reaction process of the ring opening step.

In certain embodiments R¹ is an amino acid.

In some embodiments R¹ is α-, β- or γ-amino acid, particularly an α-amino acid, more particularly a hydrophobic amino acid with short a hydrocarbon chain.

In some embodiments R¹ is alanine, valine, leucine or isoleucine.

In certain embodiment R¹ is a branched or linear, unsubstituted or substituted C₂-C₁₀ alkyl. In certain embodiments R¹ is a branched or linear, unsubstituted or substituted C₂-C₆ alkyl. In some embodiments R¹ is a branched alkyl.

In certain embodiment R¹ is a branched or linear C₂-C₁₀ alkyl. In certain embodiments R¹ is a branched or linear C₂-C₆ alkyl.

In some embodiments R¹ is - C(CH₃)₃.

In certain embodiments R¹ is in a form of alkyl-R², wherein alkyl is C₁-C₆ alkyl and R² is selected from -H, -OH, -NHR³ or -N(R³)₂, wherein R³ is an unsubstituted or substituted C₁-C₆ alkyl, particularly an unsubstituted alkyl, particularly a C₁-C₃ alkyl, more particularly a C₁ alkyl.

In certain embodiments R¹ is in a form of alkyl-R², wherein alkyl is C₁-C₆ alkyl and R² is selected from -OH, -NHR³ or -N(R³)₂, wherein R³ is an unsubstituted or substituted C₁-C₆ alkyl, particularly an unsubstituted alkyl, particularly a C₁-C₃ alkyl, more particularly a C₁ alkyl.

In certain embodiments R¹ is in a form of alkyl-R², wherein alkyl is C₁-C₆ alkyl and R² is selected from -NHR³ or -N(R³)₂, wherein R³ is an unsubstituted or substituted C₁-C₆ alkyl, particularly an unsubstituted alkyl, particularly a C₁-C₃ alkyl, more particularly a C₁ alkyl.

In certain embodiments R¹ is in a form of alkyl-R², wherein alkyl is C₁-C₆ alkyl and R² is -N(R³)₂, wherein R³ is an unsubstituted or substituted C₁-C₆ alkyl, particularly an unsubstituted alkyl, particularly a C₁-C₃ alkyl, more particularly a C₁ alkyl.

In certain embodiments R¹ is in a form of aromatic group, wherein the aromatic group is phenyl or a substituted phenyl.

In certain embodiments R¹ is in a form of aromatic group, wherein the aromatic group is a substituted phenyl, wherein the substituent is NHR³ or -NR³₂, wherein R³ is an unsubstituted or substituted C₁-C₆ alkyl, particularly an unsubstituted alkyl, particularly a C₁-C₃ alkyl, more particularly a C₁ alkyl.

In certain embodiments R¹ is in a form of aromatic group, wherein the aromatic group is a substituted phenyl, wherein the substituent is -NR³₂, wherein R³ is an unsubstituted or substituted C₁-C₆ alkyl, particularly an unsubstituted alkyl, particularly a C₁-C₃ alkyl, more particularly a C₁ alkyl.

In certain embodiments R¹ is in a form of a moiety of a formula (R⁵)(R⁶)(R⁷)C-(C=O)-O-, with each of R⁵, R⁶ or R⁷ being selected independently from H, a C₁-C₆ alkyl, particularly a C₁-C₃ alkyl, -OH, -NHR³ or -NR³₂, wherein R³ is an unsubstituted or substituted C₁-C₆ alkyl, particularly an unsubstituted alkyl, particularly a C₁-C₃ alkyl, more particularly a C₁ alkyl , wherein in particularly in case of one of R⁵, R⁶ or R⁷ is -OH, NHR³ or -NR³₂ the other two are selected from -H or C₁-C₆ alkyl, particularly a C₁-C₃ alkyl.

In certain embodiments R¹ is in a form of a moiety of a formula (R⁵)(R⁶)(R⁷)C-(C=O)-O-, with each of R⁵, R⁶ or R⁷ being selected independently from H, a C₁-C₆ alkyl, particularly a C₁-C₃ alkyl, -OH, -NHR³ or -NR³₂, wherein R³ is an unsubstituted or substituted C₁-C₆ alkyl, particularly an unsubstituted alkyl, particularly a C₁-C₃ alkyl, more particularly a C₁ alkyl , wherein at least one of R⁵, R⁶ or R⁷ is -OH, NHR³ or -NR³₂ and the other two are selected from -H or C₁-C₆ alkyl, particularly a C₁-C₃ alkyl.

In certain embodiments R¹ is in a form of a moiety of a formula (R⁵)(R⁶)(R⁷)C-(C=O)-O-, with each of R⁵, R⁶ or R⁷ being selected independently from a C₁-C₆ alkyl, particularly a C₁-C₃ alkyl, -OH, or NHR³, -NR³₂, wherein R³ is an unsubstituted or substituted C₁-C₆ alkyl, particularly an unsubstituted alkyl, particularly a C₁-C₃ alkyl, more particularly a C₁ alkyl , wherein in particularly in case of one of R⁵, R⁶ or R⁷ is -OH, NHR³ or -NR³₂ the other two are selected from -H or C₁-C₆ alkyl, particularly a C₁-C₃ alkyl.

In certain embodiments R¹ is in a form of a moiety of a formula (R⁵)(R⁶)(R⁷)C-(C=O)-O-, with each of R⁵, R⁶ or R⁷ being selected independently from a C₁-C₆ alkyl, particularly a C₁-C₃ alkyl, OH or -NR³₂, wherein R³ is an unsubstituted or substituted C₁-C₆ alkyl, particularly an unsubstituted alkyl, particularly a C₁-C₃ alkyl, more particularly a C₁ alkyl , wherein in particularly in case of one of R⁵, R⁶ or R⁷ is -OH or -NR³₂ the other two are selected from -H or C₁-C₆ alkyl, particularly a C₁-C₃ alkyl.

In certain embodiments R¹ is in a form of a moiety of a formula (R⁵)(R⁶)(R⁷)C-(C=O)-O-, with each of R⁵, R⁶ or R⁷ being selected independently from a C₁-C₆ alkyl, particularly a C₁-C₃ alkyl or -NR³₂, wherein R³ is an unsubstituted or substituted C₁-C₆ alkyl, particularly an unsubstituted alkyl, particularly a C₁-C₃ alkyl, more particularly a C₁ alkyl , wherein in particularly in case of one of R⁵, R⁶ or R⁷ is -NR³₂ the other two are selected from -H or C₁-C₆ alkyl, particularly a C₁-C₃ alkyl.

In certain embodiments, the ring opening step is performed in the presence of a base metal, in particular Zn, and a strong acid.

In certain embodiments, the ring opening step is performed in the presence of a base metal, in particular Zn, and a strong acid, wherein the strong acid is selected from HCI, HBr, H₂SO₄, HNO₃, more particularly the strong acid is HCI.

In certain embodiments, the ring opening step is performed in the presence of a base metal, in particular Zn, and HCI.

In certain embodiments, the ring opening step is performed in the presence of Zn and HCI.

In certain embodiments the ring opening step is performed an organic, polar or nonpolar solvent.

In certain embodiments the ring opening step is performed in toluene or an alcohol, particularly a C₁-C₆ alcohol, or a mixture thereof.

In certain embodiments the ring opening step is performed in toluene or an alcohol, particularly a C₁-C₄ alcohol, or a mixture thereof.

In certain embodiments the ring opening step is performed in a mixture of nonpolar and polar solvents, wherein the ratio between the nonpolar and polar solvents is in between 3:1 to 1:10, more particularly between 1:1 to 1:3.

In certain embodiments the ring opening step is performed in a mixture toluene and alcohol, wherein the ratio between toluene and the alcohol is in between 3:1 to 1:10, more particularly between 1:1 to 1:3.

In certain embodiments the ring opening step is performed in a mixture toluene and C₂-C₄ alcohol, wherein the ratio between toluene and the alcohol is in between 3:1 to 1:10, more particularly between 1:1 to 1:3.

In certain embodiments the ring opening step is performed in a mixture of toluene and ethanol or toluene and propanol, wherein the ratio between toluene and the alcohol is in between 3:1 to 1:10, more particularly between 1:1 to 1:3.

In certain embodiments R⁴ is selected from -F, a perfluorinated C₁-C₆ alkyl, C₁-C₆ alkyl, or C₁-C₆ alkyl phenyl. In some embodiments R⁴ is selected from -F, a perfluorinated C₁-C₃ alkyl, C₁-C₃ alkyl, or C₁-C₃ alkyl phenyl. In some embodiments R⁴ is selected from -F, methyl, trifluoromethyl, methyl phenyl.

In certain embodiments the leaving group X is a halogen or OSO₂R⁴

In certain embodiments the leaving group X is a halogen.

In certain embodiments the leaving group X is Cl.

In certain embodiments the reducing agent is metal hydride or an organometal hydride.

In some embodiments the reducing agent is aluminum hydride or a boron hydride or mixtures or complexes containing aluminum hydride or boron hydride.

In some embodiments the reducing agent is LiAlH₄, Red-AI (Natrium-bis(2-methoxyethoxy)-aluminum hydride) or DIBAL (Diisobutylaluminum hydride) or LiBHEt₃.

In certain embodiments the leaving group Y is selected from OH, dinitrogen, dialkyl ether, halogen, alkylcarboxylates, perfluoroalkylsulfonates, alkylsulfonates, nitrate, phosphate, amide.

In certain embodiments the leaving group Y is a halogen.

In certain embodiments the leaving group Y is Cl.

A second aspect of the invention are intermediate products of the reaction chain above in form of compounds of general formula (I), formula (II) or formula (III), particularly formula (II) or formula (III), 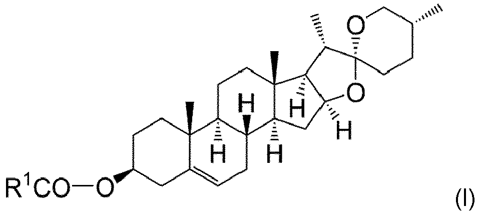
- wherein R¹ has the same definition as above with the exception that R¹ is not an unsubstituted alkyl 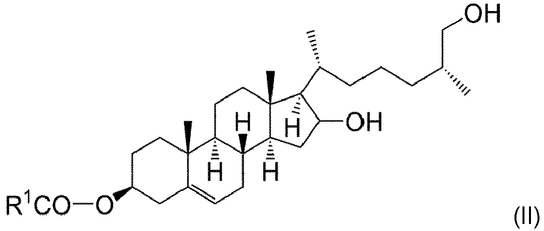
- wherein R¹ has the same definition as above, 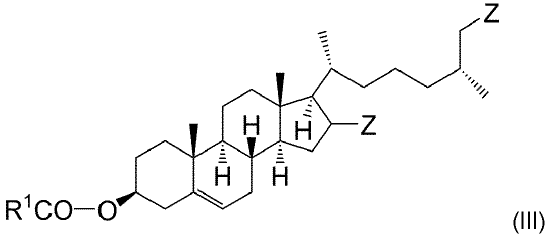
- wherein R¹ and Z have the same definition as above.

These novel compounds can be synthesised in high yields in the abovementioned reactions. Their unique features make them suitable for efficient synthesis of various steroids.

### Methods

One potential route is described in scheme 1:

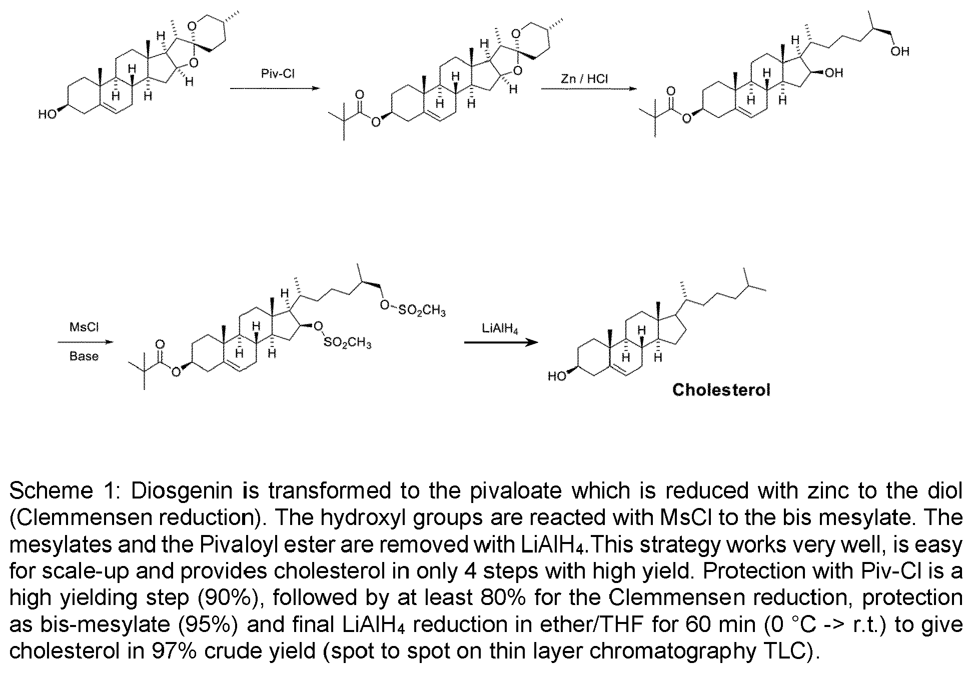

### Examples Scheme 1

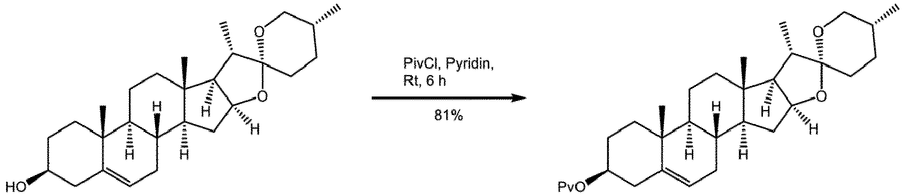

13 g of diosgenin was dissolved in 110 ml of pyridine and 4,6 ml of pivaloyl chloride was dropped in. The reaction was mixed for 6 hours in room temperature. After the completion, the reaction was diluted in 250 ml of toluene. Organic phase was washed with 1 M HCI solution (3 x 150 ml), saturated NH₄Cl solution (1 x 100 ml), water (1 x 100 ml) and saturated NaCl. The solution was dehydrated over Na₂SO₄. The product was then repeatedly crystalized, washed and redissolved to increase its purity. The reaction yielded 12.66 g of piv-diosgenin.

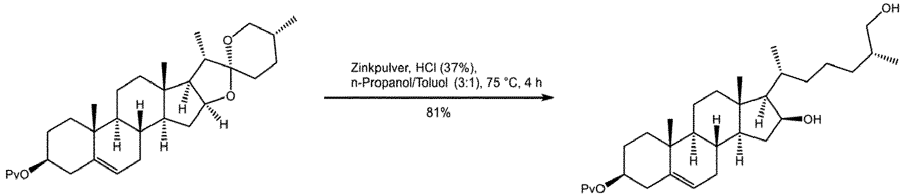

The entire Piv-diosgenin and 250 g of zinc powder were suspended in 0.5 l of n-propanol/toluene (3:1) and warmed up to 75°C. Hydrochloric acid (37%) was dropped in and the reaction was conducted for 3 hours in 80°C. The solids were separated from liquid and washed with toluene and water. The phases were separated, and the organic phase was washed with water (2 x 150ml). Combined phases was extracted with toluene (1 x 100 ml). Combined organic phase was dehydrated over Na₂SO₄. Product was crystalized overnight. The product was then repeatedly crystalized, washed and redissolved to increase its purity, yielding 10.33 g piv-diosgenin-diol.

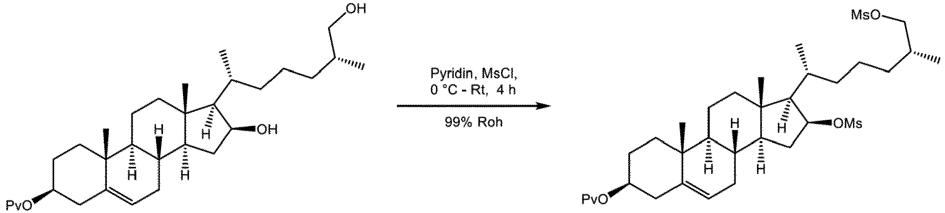

10.33 g of piv-diosgenin-diol was added to 100 ml of pyridine. 4.75 ml of methanesulfonyl chloride was slowly added dropwise. The reaction mixture was stirred at room temperature overnight. The reaction mixture was then diluted with 300 ml of ethyl acetate, washed with 1 M HCI (3 x 200 ml), saturated NH₄Cl solution (1 x 200), saturated NaCl solution and dried over Na₂SO₄. The solvent was removed on a rotary evaporator and crude product in the form of a white foam was obtained.

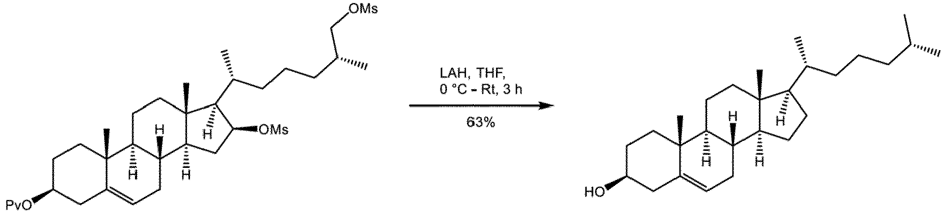

4 g (5 equivalents) of lithium aluminum hydride (LAH) were suspended in 50 ml of dry tetrahydrofuran and cooled to 0°C. Piv-diosgenin mesylate was dissolved in 50 ml of dehydrated tetrahydrofuran and slowly added dropwise. The reaction mixture was stirred at RT for 3 h. After no more starting material is detected, the mixture was cooled down to 0°C and excess LAH was carefully quenched with water (30 ml). The solution was then diluted with ethyl acetate (300 ml) and water (200 ml) and the phases are separated. The organic phase was washed with saturated NH₄Cl solution (1 x 100 ml), saturated NH₄HCO₃ solution (1 x 100 ml), water (1 x 100 ml), saturated NaCl solution and dried over Na₂SO₄. The ethyl acetate was removed on a rotary evaporator. The crude product was dissolved under reflux in 50 ml of MTBE / ethanol solvent mixture (1: 2) and left to stand at RT overnight. Cholesterol crystallizes as silky-glossy flakes.

An alternate route is described in Scheme 2.

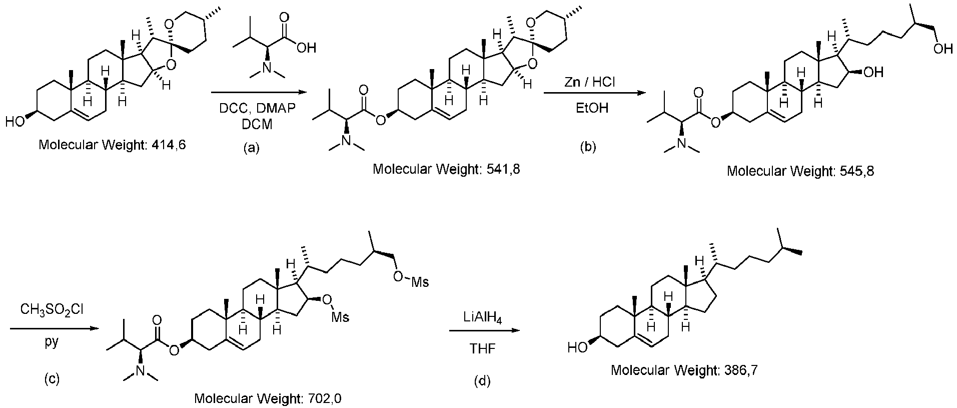

### Examples Scheme 2

N,N-Dimethyl-L-valine (251 mg, 1,2 eq.) in DCM (10 ml) was stirred with dicyclohexylcarbodiimide (386 mg, 1.3 eq.) 17 min at room temperature and subsequently diosgenin (517 mg, 1.0 eq.) and 4-N,N-dimethylaminopyridine (35mg, 0.2 eq.) were added. After 1 h, the mixture was washed twice with water, dried over MgSO₄ and evaporated. The residue was purified by flash chromatography on silica (pentane/ether 4:1) to obtain the ester (452 mg, 61%).

A suspension of zinc (7.6 g) and the ester (405 mg) in ethanol (26 ml) was heated to reflux and hydrochloric acid (37%, 8 ml) was added over a period of 1 h. After additional 2 h at reflux, the remaining zinc was filtered off, the filtrate treated with water and extracted with toluene. After drying over MgSO₄ and evaporation, the desired diol (147 mg, 36%) was isolated by flash chromatography on silica (pentane/ether 3:1).

A solution of the diol (147 mg) in pyridine (1.35 ml) was cooled to 0°C and methanesulfonyl chloride (0.23 ml, 4 eq.) was added. After 1 h at ambient temperature, the mixture was diluted with ethyl acetate and subsequently washed with 1M aq. HCI, saturated aq. NaHCO₃, and brine. The solution was dried (MgSO₄) and evaporated to give the crude bis-mesylate (215 mg), which was used for the next step.

Crude bis-mesylate (215 mg) was dissolved in THF (3 ml) and added at room temperature to a suspension of LiAlH₄ (17 mg, 1.5 eq.) in THF (3 ml). After 1 h, another portion of LiAlH₄ (17 mg, 1.5 eq.) was added and the mixture was stirred for an additional hour. The reaction mixture was first quenched with 1M aq. NaOH and then divided between aq. Na-K-tartrate and ethyl acetate. The organic layer was dried (MgSO₄) and evaporated. Cholesterol (47 mg, 45% over two steps) was isolated by flash chromatography on silica (pentane/diethyl ether 2:1).

Other alternative routes are via the protecting group m-(N,N-Dimethylamino)benzoic Acid or a-Hydroxy-*iso*-butyric Acid.

### m-(N,N-Dimethylamino)benzoic Acid Route

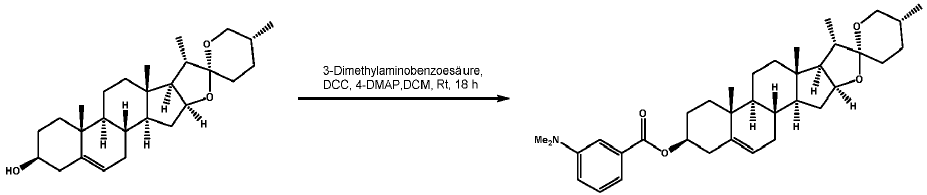

1 g diosgenin, 0.5 g DCC, 0.2 g 4-DMAP and 0.4 g of m-(N,N-dimethylamino)benzoic acid were stirred in 15 ml of try DCM for 18 hours at room temperature. The mixture was diluted with 20 ml of DCM and was washed with saturated NaHCO₃ solution, saturated NH₄Cl solution, water, and brine. The organic layer was dried over MgSO₄ and the solvent was removed under reduced pressure and the crude product was dried under HV.

### α-Hydroxy-iso-butyric Acid Route

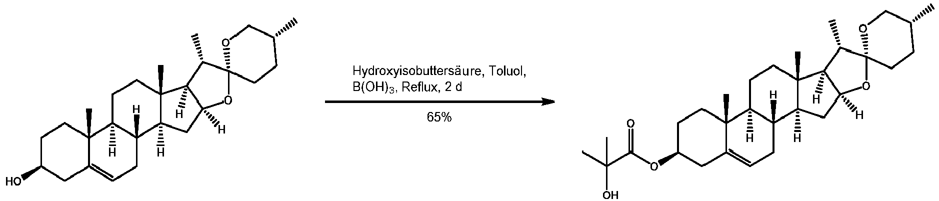

5g diosgenin, 40 mg B(OH)₃ and 1.5 g of α-hydroxy-*iso*-butyric acid was dissolved in 50 ml of toluene and was refluxed for 2 days with a Stork-Dean-trap. The mixture was diluted with 50 ml of toluene and was washed with saturated NaHCO₃ solution, saturated NH₄Cl solution, water, and brine. The organic layer was dried over MgSO₄ and the solvent was removed under reduced pressure. The crude product was flash chromatographed (pentene/ether 1:1) and 3.9 g of a white powder was obtained.

## Claims

1. A synthesis method of cholesterol comprising:
a) a ring opening step, wherein the compound of formula (I) 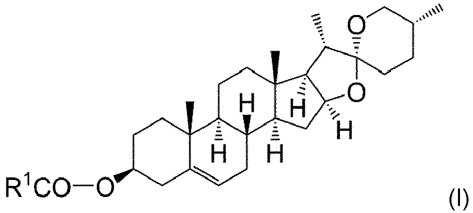 is reacted in a Clemmensen reaction or a variant thereof, yielding compound of formula (II); 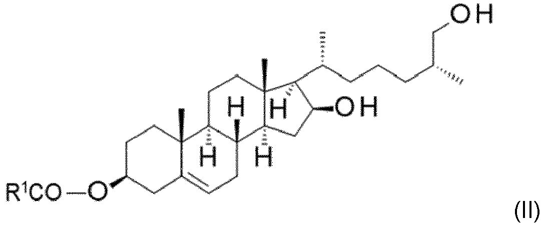
b) an activation step with SOCI2, PBr₃ R⁴SO₂X, yielding a compound of formula (III), preferably (IIIa); 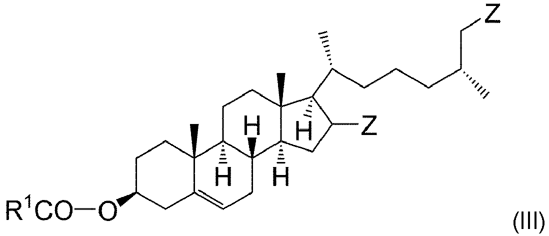 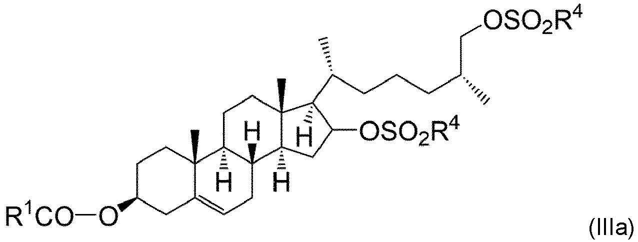
c) a reduction step, wherein compound (III) is reacted with a reducing agent, yielding cholesterol (IV) 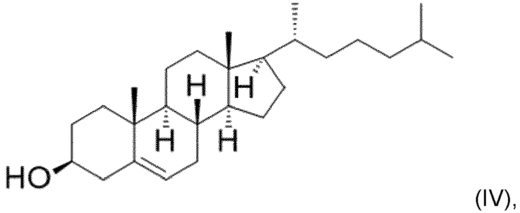 wherein
- R¹ is selected from unsubstituted or substituted alkyl, amino acid, cyclic alkyl, particularly a C₂-C₆ cyclic alkyl, or alkyl-R², heterocycle, an aromatic or heteroaromatic group, particularly R¹ is selected from unsubstituted or substituted alkyl, amino acid, cyclic alkyl, particularly a C₂-C₆ cyclic alkyl, or alkyl-R², more particularly from unsubstituted or substituted alkyl, amino acid or alkyl-R²,
- R² is selected from H, -OH, -CN, -NH₂, -NHR³ or -NR³₂,
- R³ is an unsubstituted or substituted C₁-C₆ alkyl,
- R⁴ is a substituted or unsubstituted alkyl, cyclic alkyl or aromatic group,
- X is a leaving group or OSO₂R⁴
- Z is -CI, -Br or -OSO₂R⁴, preferably -OSO₂R⁴.

2. The method according to claim 1, wherein compound (I) is provided by a reaction of diosgenin (V) 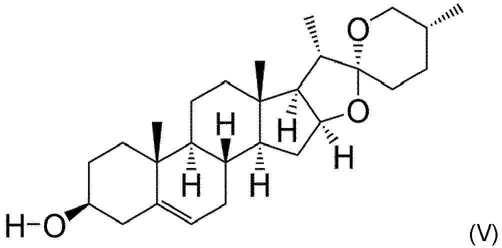 with compound of a formula R¹COY, wherein R¹ has the same meaning as defined above and Y is a leaving group, wherein in particular the leaving group is selected from halogen, particularly CI.

3. The method according to any of the previous claims, wherein the amino acid of R¹ is selected from α-, β- or γ-amino acid, particularly α-amino acid, more particularly alanine, valine, leucine, isoleucine.

4. The method according to any of the previous claims, wherein the alkyl of R¹ is a unsubstituted or substituted C₂-C₁₀ alkyl, more particularly a unsubstituted or substituted C₂-C₆ alkyl, even more particularly -C(CH₃)₃, wherein in particular the alkyl are branched.

5. The method according to any of the previous claims, wherein the alkyl-R² of R¹ is defined by a C₁-C₆ alkyl for the alkyl and R² is selected from H, -OH, -NHR³ or -NR³₂, particularly from -OH, -NHR³ or -NR³₂, more particularly from NHR³ or -NR³₂, even more particularly R² is -NR³₂, wherein R³ is an unsubstituted or substituted C₁-C₆ alkyl, particularly a C₁-C₃ alkyl, more particularly a C₁ alkyl.

6. The method according to any of the previous claims, wherein the aromatic group of R¹ is phenyl or a substituted phenyl, particularly a substituted phenyl, wherein the substituent is selected from NHR³ or -NR³₂, particularly from -NR³₂, wherein R³ is an unsubstituted or substituted C₁-C₆ alkyl, particularly a C₁-C₃ alkyl, more particularly a C₁ alkyl.

7. The method according to any of the previous claims, wherein R¹ is (R⁵)(R⁶)(R⁷)C-(C=O)-O-, with each of R⁵, R⁶ or R⁷ being selected independently from H, a C₁-C₆ alkyl, particularly a C₁-C₃ alkyl, -OH, -NHR³ or -NR³₂, particularly from a C₁-C₆ alkyl, particularly a C₁-C₃ alkyl, -OH, or NHR³, -NR³₂, more particularly from a C₁-C₆ alkyl, particularly a C₁-C₃ alkyl, OH or -NR³₂, even more particularly from a C₁-C₆ alkyl, particularly a C₁-C₃ alkyl or -NR³₂ wherein particularly in case of one of R⁵, R⁶ or R⁷ is selected from -OH, NHR³ or -NR³₂ the other two are selected from H, a C₁-C₆ alkyl, particularly a C₁-C₃ alkyl, wherein R³ is an unsubstituted or substituted C₁-C₆ alkyl, particularly a C₁-C₃ alkyl, more particularly a C₁ alkyl.

8. The method according to to any of the previous claims, wherein the ring opening step is performed in the presence of a base metal, in particular Zn, and a strong acid, wherein in particular the strong acid is selected from HCI, HBr, H₂SO₄, HNO₃, more particularly the strong acid is HCI.

9. The method according to any of the previous claims, wherein ring opening step is performed in an organic, polar or nonpolar solvent, particularly toluene or an alcohol, particularly an C₁-C₆ alcohol, or a mixture thereof.

10. The method according to claim 9, wherein the solvent is a mixture of nonpolar and polar solvents, particularly toluene/alcohol, more particularly toluene/C₂-C₄ alcohol, even more particularly toluene/ethanol or toluene/propanol, wherein the ratio between the nonpolar and polar solvents, particularly toluene/alcohol, is in between 3:1 to 1:10, more particularly between 1:1 to 1:3.

11. The method according to any of the previous claims, wherein R⁴ is selected from -F, a perfluorated C₁-C₆ alkyl, C₁-C₆ alkyl, or C₁-C₆ alkyl phenyl, particularly -F, a perfluorated C₁-C₃ alkyl, C₁-C₃ alkyl, or C₁-C₃ alkyl phenyl, more particularly -F, methyl, trifluoromethyl, methyl phenyl.

12. The method according to any of the previous claims, wherein the leaving group of X is selected from halogen, particularly Cl.

13. The method according to any of the previous claims, wherein reducing agent is metal hydride or organometal hydride, particularly an aluminum hydride or a boron hydride or mixtures or complexes containing aluminum hydride or boron hydride, more particularly the reducing agent is LiAlH₄, Red-AI,DIBAL or LiBHEt₃.

14. The method according to any of the claims 2 to 13, wherein the leaving group Y is selected from OH, dinitrogen, dialkyl ether, halogen, alkylcarboxylates, perfluoroalkylsulfonates, alkylsulfonates, nitrate, phosphate, amide, particularly halogen, even more particularly chloride.

15. A compound of general formula (I), formula (II) or formula (III), particularly (IIIa) in particular formula (II) or formula (III), particularly (IIIa), 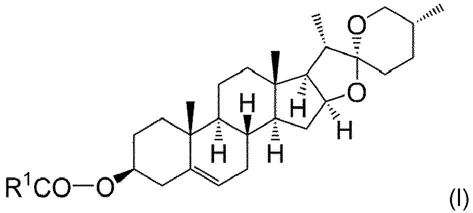
- wherein R¹ has the same definition as above with the exception that R¹ is not an unsubstituted alkyl 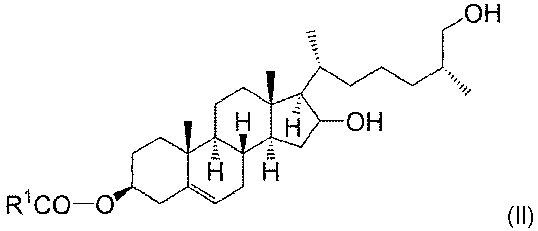
- wherein R¹ has the same definition as above, 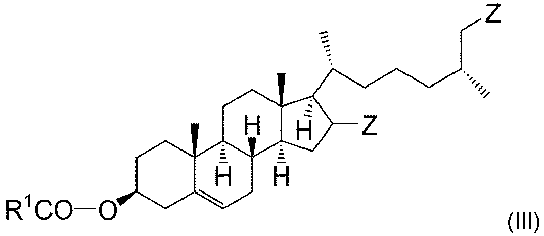 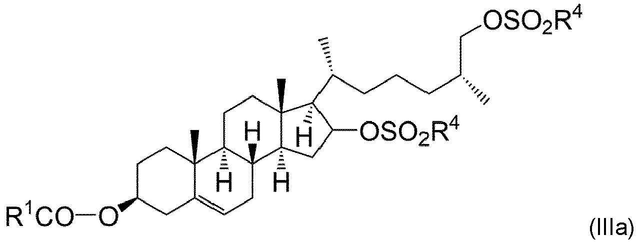
- wherein R¹, R⁴ and Z have the same definition as above.
